(19) Europäisches Patentamt
European Patent Office
Office européen des brevets

(11) **EP 1 684 615 B1**

(12) **EUROPEAN PATENT SPECIFICATION**

(45) Date of publication and mention
of the grant of the patent:
**24.02.2010 Bulletin 2010/08**

(51) Int Cl.:
***A47C 21/06*** *(2006.01)*    ***A61F 5/48*** *(2006.01)*

(21) Application number: **04798649.2**

(22) Date of filing: **22.11.2004**

(86) International application number:
**PCT/GB2004/004942**

(87) International publication number:
**WO 2005/051134 (09.06.2005 Gazette 2005/23)**

(54) **MATTRESS PROTECTION**

MATRATZENSCHUTZ

PROTECTION DE MATELAS

(84) Designated Contracting States:
**AT BE BG CH CY CZ DE DK EE ES FI FR GB GR
HU IE IS IT LI LU MC NL PL PT RO SE SI SK TR**

(30) Priority: **21.11.2003 GB 0327138**

(43) Date of publication of application:
**02.08.2006 Bulletin 2006/31**

(73) Proprietor: **UCL Business PLC
97 Tottenham Court Road
London W1T 4TP (GB)**

(72) Inventors:
• **BAIN, Duncan
Aberdeen,
Scotland (GB)**
• **PELL, Martin, Ferguson
Chesham Bois,
Buckinghamshire HP6 5NP (GB)**

• **NICHOLSON, Graham
Middlesex HA7 4HE (GB)**

(74) Representative: **Maughan, Sophie Louise et al
Scott & York
Intellectual Property Ltd
45 Grosvenor Road
St Albans, Hertfordshire AL1 3AW (GB)**

(56) References cited:
**EP-A- 1 138 293    WO-A-94/10958
US-A- 5 137 033    US-A- 5 291 181
US-B1- 6 341 393**

• **PATENT ABSTRACTS OF JAPAN vol. 1998, no.
05, 30 April 1998 (1998-04-30) & JP 10 020789 A
(KAMIYAMA KISHIKO), 23 January 1998
(1998-01-23)**

## Description

### Field of the Invention

The present invention concerns improvements in and relating to mattress protectors and provides methods and means for monitoring the integrity of a mattress protector to determine if bodily fluids have penetrated through to the mattress.

### Background to the Invention

Hospital mattresses form the platform for delivering routine care to patients. In recent years increasing attention has been paid to their specifications, including their ability to improve comfort for patients, prevent pressure ulcers, appropriately stabilise patients with fractures or following surgery, reduce the risk of rolling off the mattress, and facilitate nursing management of the patients including toileting. A further requirement is that mattress covers be impenetrable to body fluids so that the inner core, which usually comprises a composite of foams, does not become contaminated. Once the integrity of the cover has been breached (technically known as "strike-through"), the potential for the mattress core to become a medium for the growth of bacteria and fungal spores is high. The combined presence of moisture, infectious agents and the warmth continuously generated by the multiple users of the mattress is an ideal incubation environment for pathogens.

In September 1999 the Medical Devices Agency issued a Safety Notice (MDA SN1999 (31) alerting hospitals of the hazards to staff and patients associated with mattresses in poor condition. In recent years procedures for supplying and servicing mattresses in hospitals have changed. Traditionally nursing staff have ordered, cleaned and audited the condition of mattresses, but with increasing pressure on their time, non-patient contact activities such as mattress management are being increasingly devolved to non-nursing staff. The UK is leading Europe in the implementation of "total mattress management" and "total bed management" contracts where the manufacturer/distributors of hospital mattress are contracted to supply mattresses against clinical and technical specifications. These specifications are led by the NHS Purchasing and Supply Agency (PASA).

Under these arrangements contractors undertake the responsibility of not only supplying hospital mattresses but also auditing their condition, ensuring that worn or leaking mattresses are withdrawn and replaced. At present this entails technicians undertaking a "sweep" through the hospital inspecting mattresses individually, physically checking for deterioration of the mechanical support characteristics of the mattress core, and inspecting for evidence of "strike-through". This is a very unpleasant process and may also represent a potential health and safety risk to staff undertaking the audits.

Technology exists for monitoring soiling of sheets such as draw sheets that cover mattresses as exemplified in US-6,341,393 (Votel).

This invention seeks as one objective to provide a new technology to enable mattress audit technicians or hospital staff to determine whether a mattress cover has leaked at any time in the mattress' service, without removing the cover. Avoiding the necessity of removing the mattress cover both simplifies the task and reduces the risk of infections spreading.

### Summary of the Invention

According to a first aspect of the present invention there is provided a mattress protector to shield a mattress or mattress core from body fluids, the protector comprising a shielding cover for fitting over the mattress or mattress core, the shielding cover having a layer that is impermeable to body fluids that is outermost in use, and a detector within the cover or below or in an under layer of the cover, the detector being below the impermeable layer of the shielding cover, to detect body fluid that has passed into or through the cover, the fluid having penetrated the impermeable layer of the shielding cover. The protector may be separate from or integral with a mattress and, in the latter case, may comprise all or part of the casing that envelopes the mattress core. The substantially liquid-impermeable cover is preferably breathable.

In a first preferred embodiment of the invention the cover is a transparent cover for a mattress with an inter-layer or under-layer, suitably an absorbent under-layer, having a dye that is activated by a body fluid to provide a visual indication of presence of the body fluid. The inter- or under-layer is preferably impregnated with the dye.

The dye is preferably unaffected by water vapour. However, if body fluid such as urine penetrates through the mattress, indicators in the dye will show through the transparent cover. An indicator specific to urine and unaffected by water (many of which are commercially available and are used, for example, to expose urination in swimming pools) is advantageous, since water vapour may be present in the mattress during correct functioning of the cover, where the cover is specified to be "breathable", i.e. transmits water vapour.

Preferably, the absorbent layer is made from a stretchable material, suitably crepe or similar, in order to minimise perturbation of the cover stretch properties. Alternatively, incisions could be made in the absorbent sheet, similar to those in lattice pastry or expanded sheet metal, so as to allow stretch in the sheet material.

In a second preferred embodiment of the invention, instead of having a dye-based detector the detector comprises an apparatus that monitors electrical conductivity. This suitably uses electrically conductive threads or fibres such as, for example, an electrically conductive yarn (eg, silver plated nylon), forming rows or a matrix over the inter- or under-layer. Suitably the threads form the warp or weft of a thin flexible woven under- layer.

Preferably the mattress in this second embodiment is fitted with a small processor, preferably a micro-processor, which monitors the electrical conductivity between neighbouring conductive threads in the sensing layer and records any change.

In the event of a leak of ionic (electrically conductive) fluid, as all body fluids are, a short circuit between threads will be detected and recorded. The micro-processor may have a very low power consumption and could be battery-powered and be interrogated using an inductive link by an external device to determine if any leak events had occurred.

## Brief Description of the Drawings

The preferred embodiments of the present invention will now be more particularly described by way of example with reference to the accompanying drawings, wherein:

Figure 1 is a sectional view of an end of a mattress encased in the mattress protector of the first preferred embodiment;

Figure 2 is an isometric view of a mattress encased in a mattress protector of the second preferred embodiment, showing the outer cover part cut-away to view the matrix of electrically conductive yarn that underlies the outer cover;

Figure 3 is a schematic circuit diagram for the electrical conductivity-based detector of the second preferred embodiment;

Figure 4 is a further schematic circuit diagram for the electrical conductivity-based detector of the second preferred embodiment showing an example suitable geometry of the positive and negative tracks of the detector;

Figure 5 is a photographic image of a first example of electrical conductivity-based detector having a 14.5mm conductor repeat interval, labelled Specimen MLSF001;

Figure 6 is a photographic image of a second example of electric conductivity-based detector having a 11.6mm conductor repeat interval, labelled Specimen MLSF002;

Figure 7 is a photographic image of a third example of electrical conductivity-based detector having a 4.9mm conductor repeat interval, labelled Specimen MLSF003;

Figure 8 is a graph of change in resistance over an elapsed time period measured in seconds on wetting MLSF001 with distilled water, 1st run;

Figure 9 is a graph of change in resistance over an elapsed time period measured in seconds on wetting MLSF001 with distilled water, 2nd run;

Figure 10 is a graph of change in resistance over an elapsed time period measured in seconds on wetting MLSF001 with 4% saline solution, 1st run;

Figure 11 is a graph of change in resistance over an elapsed time period measured in seconds on wetting MLSF001 with 4% saline solution, 2nd run;

Figure 12 is a graph of change in resistance over an elapsed time period measured in seconds on wetting MLSF002 with distilled water, 1st run;

Figure 13 is a graph of change in resistance over an elapsed time period measured in seconds on wetting MLSF002 with distilled water, 2nd run;

Figure 14 is a graph of change in resistance over an elapsed time period measured in seconds on wetting MLSF002 with 4% saline solution, 1st run;

Figure 15 is a graph of change in resistance over an elapsed time period measured in seconds on wetting MLSF002 with 4% saline solution, 2nd run;

Figure 16 is a graph of change in resistance over an elapsed time period measured in seconds on wetting MLSF003 with distilled water, 1st run;

Figure 17 is a graph of change in resistance over an elapsed time period measured in seconds on wetting MLSF003 with distilled water, 2nd run;

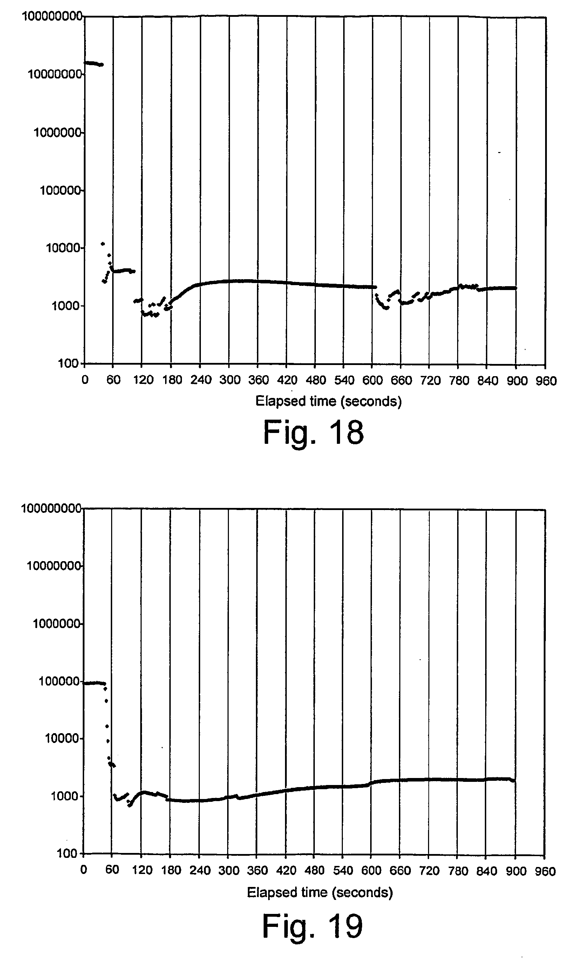

Figure 18 is a graph of change in resistance over an elapsed time period measured in seconds on wetting MLSF003 with 4% saline solution, 1st run;

Figure 19 is a graph of change in resistance over an elapsed time period measured in seconds on wetting MLSF003 with 4% saline solution, 2nd run; and

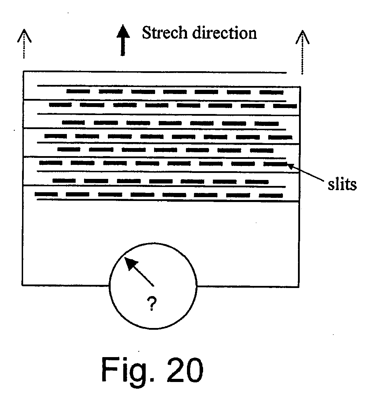

Figure 20 is a schematic circuit diagram similar to Figure 4 but in which the positive and negative conductor tracks are in an expandable sheet that is slitted between tracks to facilitate stretching of the sheet in the direction marked by the arrow.

## Description of the Preferred Embodiments

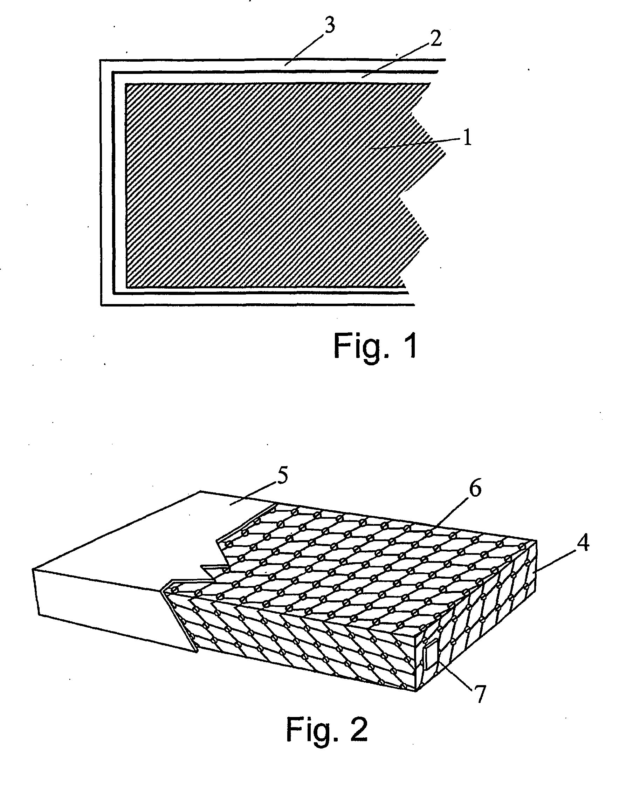

In the first preferred embodiment, illustrated in Figure 1, a transparent cover 3 is provided for the mattress 1, with an absorbent under-layer 2 impregnated with a dye that is activated by a body fluid such as urine but not by water vapour. If urine penetrates through the mattress indicators in the dye show through the transparent cover. An indicator specific to urine, or rather specific for organic compounds such as urea or glucose present in urine, is advantageous, since water vapour may be present in the mattress during correct functioning of the cover. Many mattress covers are specified to be "breathable", i.e. they transmit water vapour. Alternatively, a moisture-sensitive dye sensitive to a threshold level of moisture greater than that prevalent as water vapour may be used.

The absorbent layer is here stretchable, suitably being made from a stretchable fabric, eg a crepe-style material, or is incised/ partitioned, in order to minimise perturbation of the cover stretch properties.

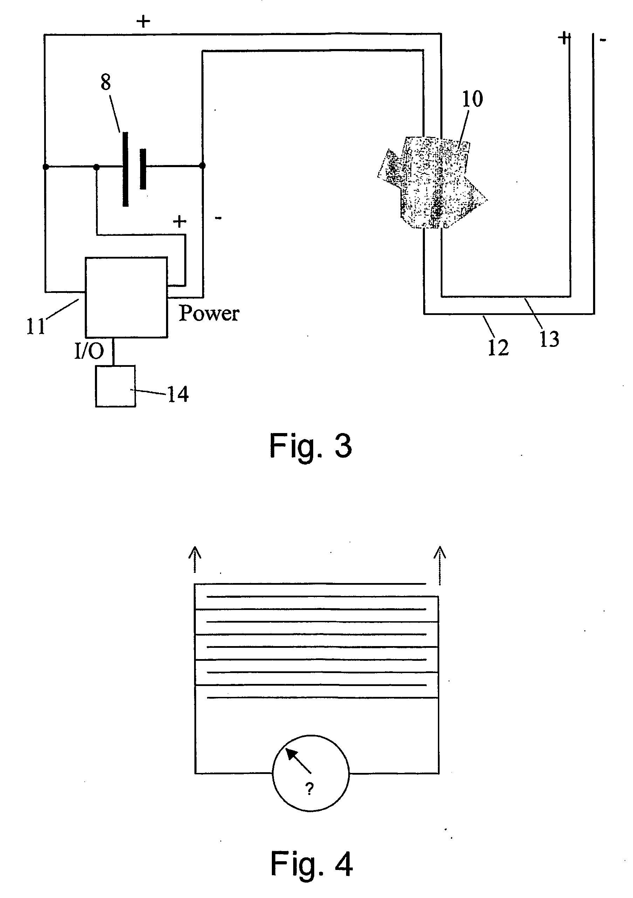

In the second preferred embodiment illustrated in Figures 2 and 3, threads of electrically conductive silver plated nylon yarn are used forming the warp or weft of a thin flexible woven under layer 6 of the mattress protector beneath the substantially liquid impermeable shielding layer 5 of the cover. The mattress 4 is fitted with a small micro-processor 7, which monitors the electrical conductivity between neighbouring conductive threads 12, 13 in the sensing layer 6. In the event of a leak of ionic (electrically conductive) fluid, a short circuit between threads is detected via the threads 12,13 and recorded by the microprocessor 7. The microprocessor 7 has a very low power consumption and is thus battery powered and entirely self-contained. It can be interrogated using an inductive link by an external device to determine if any leak events had occurred.

Referring to Figure 3, a battery 8 supplies power to the microprocessor 7 as well as supplying positive and negative threads 12 and 13 respectively. The presence of an electrolyte such as urine 10 causes a short circuit between the two threads 12 and 13, so changing the high/low state monitored at digital input 11 to the microprocessor 7. This event is stored in the memory of the microprocessor 7.

At some subsequent occasion, the assembly is interrogated by means of a radio wand, the signal of which is picked up by antenna 14. This causes the microprocessor 7 to respond with an appropriate signal to indicate whether a short has occurred at any time in the past.

This remote interrogation of the microprocessor 7 can take place using conventional RFID technology, without the necessity of removing the mattress cover 5 (so causing a spread of infection), or even the bedclothes.

In one embodiment, the circuit is completed by the presence of a leak that joins any two or more adjacent lines (weft bars of conductive yarn). The tracks of conductive yarn can be produced in various geometries to provide suitable adjacent positive and negative tracks at appropriate spacings over the mattress surface. An example of this system is illustrated in principle in figure 4.

## Experimental Examples

Sample devices were constructed as follows:

One rectangular piece of conductive-striped fabric was cut from bulk, 250 mm x 260 mm, with longer dimension weft-ways (across width).

Two rectangular pieces of fusible interfacing were cut from bulk, 250 mm x 30 mm, one fused to each shorter edge of the conductive-striped fabric.

All four edges of the assembly were over-locked. Rectangular holes were cut out of alternate weft bars one each side (with the sides offset by one bar, creating the circuit as shown above) approximately 14 mm from edge and approximately 13 mm wide. Two lengths of 17 mm wide tape with tinned-copper wire floats were cut 280 mm long. One is stitched in place on each side such that the wire floats contact the weft bars. An overhang of 30 mm is placed at one end to be used for attaching the multimeter.

The specimens were assessed for their response to distilled water and to a 4% saline solution. The former should indicate the likely behaviour if condensation builds up in the system during use, with an alarm not desirable in this circumstance. The latter should indicate the likely behaviour when urine becomes present in the system as a result of the mattress cover leaking, with an alarm to be triggered.

Therefore, it is preferable that the sensitivity of the system to distilled water will be much lower than the sensitivity to saline.

Method of Test.

The specimen to be tested was placed on a non-absorbent bench, in an unconditioned lab. The ambient temperature was approximately 19°C for all tests. The terminals at the end of each side of the specimen were connected to a Center SE122 True RMS multimeter, set to measure the resistance of the specimen. This was connected to the serial port of a laptop computer running the SE120 Testlink data collection software. The resistance of the specimen was acquired every two seconds during the test. This data was saved in CSV form (comma separated values) which was then exported to Microsoft Excel for further analysis.

Two fluids were used for the testing: distilled water and a 4% w/w saline solution (i.e. sodium chloride in distilled water). There are 'recipes' for artificial urine which can be prepared using sodium chloride, potassium chloride, sodium phosphate and urea, plus traces of other chemicals (such as amino acids) but in discussion with ACDS it was decided that the response to saline would be provide sufficient information. Future testing could easily use a more realistic formulation, or samples of actual urine.

Some initial trials indicated that a total of three drops of each fluid from a disposable pipette was an appropriate volume of fluid to add to specimens of this size. It was calculated that each drop was on average 0.033 ml in volume, so the three drops was approximately 0.1 ml. This is quite a small amount of fluid, so if a useful response can be obtained from the specimens under these conditions it is likely that the system will have the required sensitivity in the final application. The fluid was dropped onto the specimen from approximately 1 cm above the fabric surface.

The fluid was not placed onto the specimen in one dose. Three one-drop doses were applied one minute apart. This was intended to simulate the more gradual fluid penetration that is likely to occur with a mattress cover leak.

Before adding the fluid, data recording was carried out for the dry specimen for 30 seconds. The first drop of fluid was added at this stage (t = 30 seconds), with another added 60 seconds after this (t = 90 seconds), and the final drop added after a further 60 seconds (t = 150 seconds). Data was collected continuously over fifteen minutes (end of test at t = 900 seconds), but one-off readings were made ten and twenty minutes after the end of recording (t = 1500 and 2100 seconds, respectively).

The fluid was placed directly onto a conductive stripe in the fabric. This placement should represent the slowest response that the specimen could provide, as the fluid has to traverse an entire non-conductive stripe to reach an adjacent conductive stripe and trigger a change in resistance. The fastest response could be expected if the drops were placed exactly between the conductive stripes, as only half a non-conductive stripe would need to be traversed to trigger the response in this case.

Results

Each specimen was tested four times, twice with each of the fluids. The order of test was such that it was unnecessary to carry out any additional processes on the specimen after test, apart from thorough drying. The fabric was not prepared in any way before testing (such as pre-washing, solvent extraction etc.). The order of testing was as follows:

**Distilled water 1 → Distilled water 2 → Saline 1 → Saline 2**

In theory, the distilled water leaves no residue on the fabric, so any washing or rinsing after each of these tests is redundant. The second saline test is, therefore, carried out on a fabric that has a sodium chloride residue on it, but before carrying out this test it was confirmed that the resistance had increased to a high level. In the absence of moisture, the sodium chloride is non-conductive, as there is no means for ion movement (and hence conduction). If the second test involved placing the drops onto the exact same region as the previous then the effect may be equivalent to having doubled the saline solution concentration. However, each of the four tests was carried out on a different region of the specimen, with no overlap of the (previously) wetted areas.

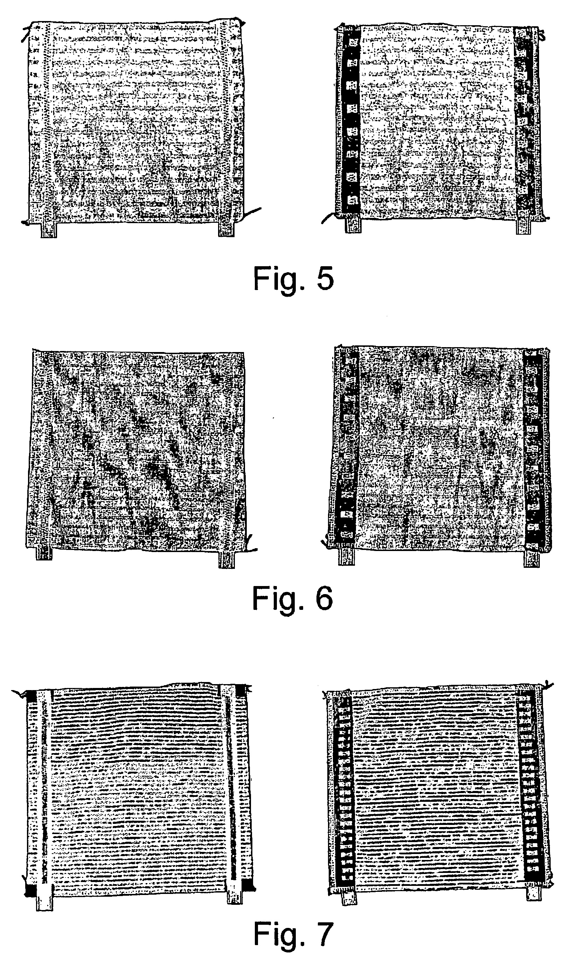

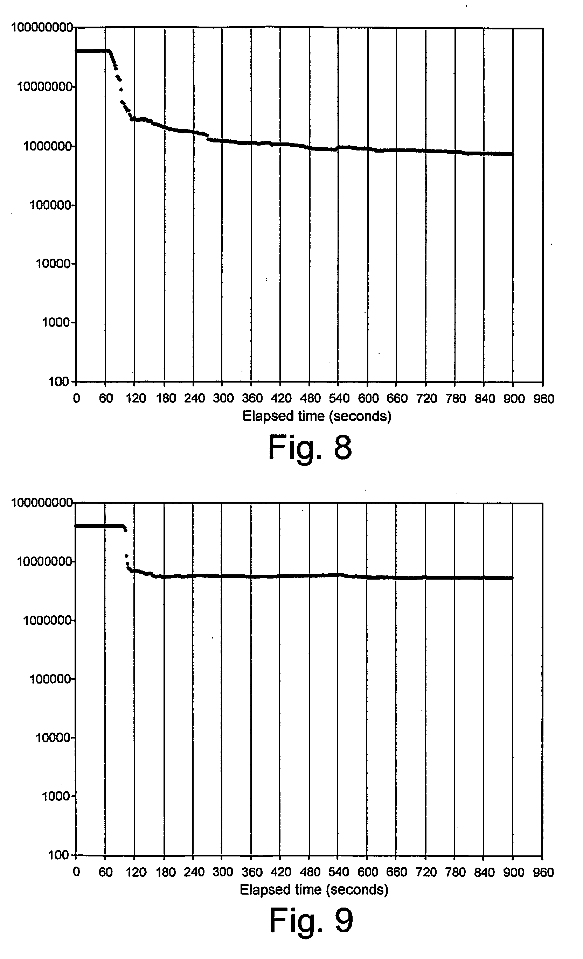

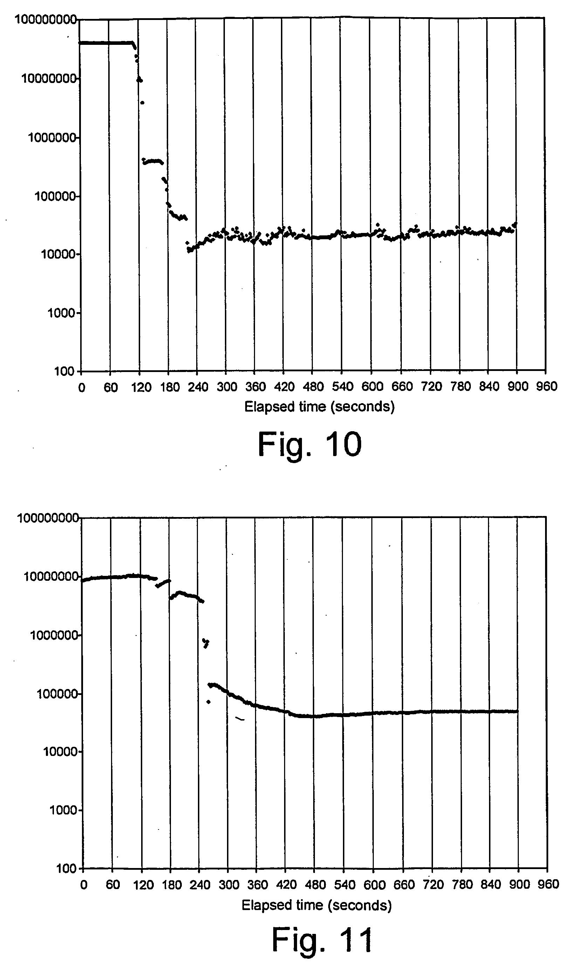

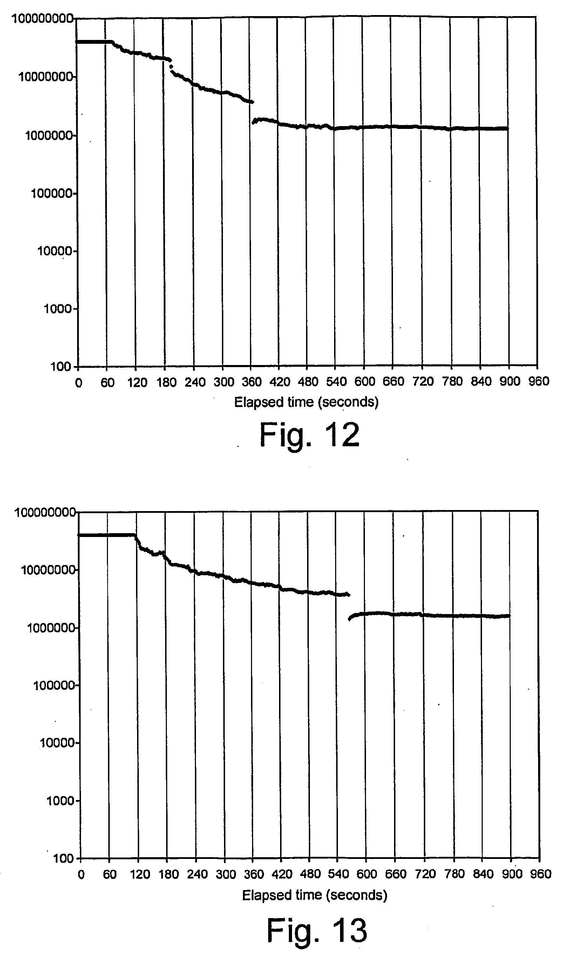

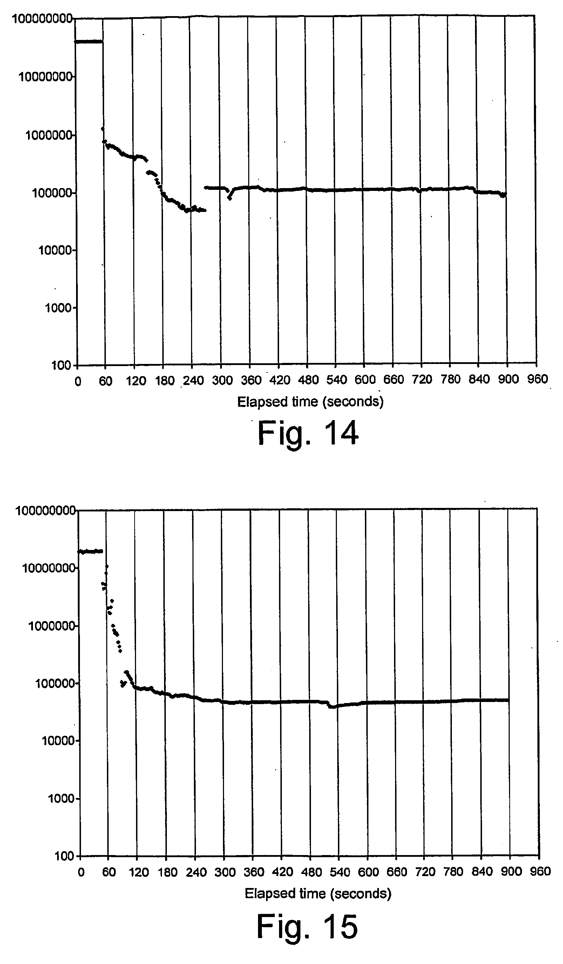

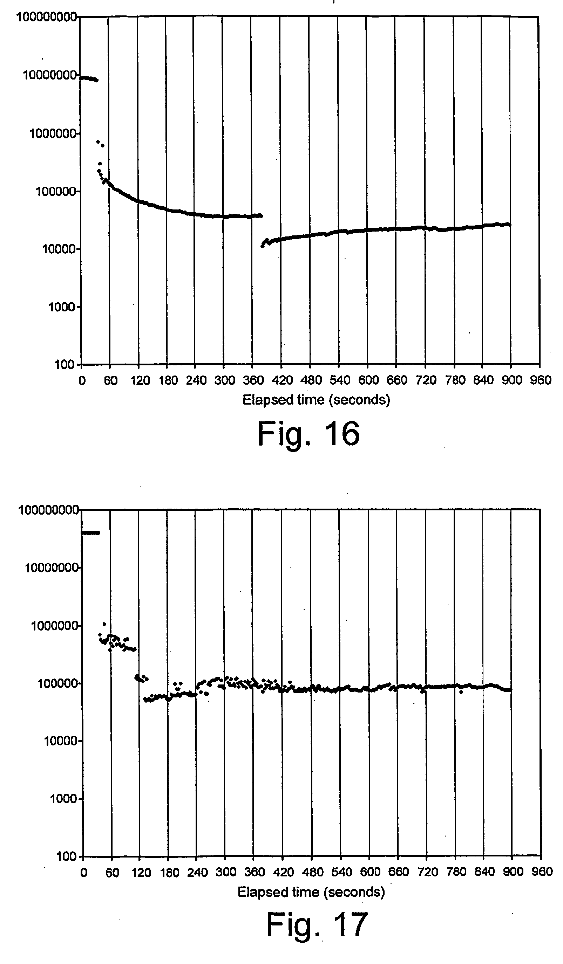

The full results for each test on each specimen are illustrated in Figures 5 to 16. A summary of the resistance values at key times is provided in Table 1. Note that the 1500 s and 2100 s readings were not made for the final assessment as the basic pattern for these readings had been established. It is also important to note that the maximum measurable resistance for the multimeter being used was 40MΩ, so readings above this limit are recorded as '> 40 M'. Where this is given as the resistance the actual resistance may be much higher - in theory (for a dry specimen with no defects) it will be in the Giga-ohm range (GΩ, $1 \times 10^9$ Ω).

Table 1. Summary of resistance changes.

| Specimen | Fluid | Test No. | Resistance at time given | | | | | |
|---|---|---|---|---|---|---|---|---|
| | | | 30 s | 90 s | 150 s | 900 s | 1500 s | 2100 s |
| MLSF001 | Distilled water | 1 | > 40 M | 13.0 M | 2.67 M | 753 k | 649 k | 639 k |
| | | 2 | > 40 M | >40M | 6.29 M | 5.36 M | 4.94 M | 4.84 M |
| | Saline solution | 1 | > 40 M | > 40 M | 390 k | 32.1 k | 67.4 k | 50.1 k |
| | | 2 | 9.36 M | 9.67 M | 9.09 M | 48.8 k | --- | --- |
| MLSF002 | Distilled water | 1 | > 40 M | 33.0 M | 23.7 M | 1.25 M | 1.28 M | 1.35 M |
| | | 2 | > 40 M | > 40 M | 19.8 M | 1.51 M | 1.39 M | 2.59 M |
| | Saline solution | 1 | >40M | 546 k | 217k | 87.0 k | 104k | 112k |
| | | 2 | 19.0 M | 105 k | 80.9 k | 48.6 k | --- | --- |
| MLSF003 | Distilled water | 1 | 8.51 M | 89.8 k | 56.8 k | 25.3 k | 117 k | 141 k |
| | | 2 | >40M | 562k | 53.8k | 75.4k | 114k | 176k |
| | Saline solution | 1 | 14.8 M | 4.21 k | 694 Ω | 2.13 k | 2.08 k | 2.32 k |
| | | 2 | 95.0 k | 1.06 k | 1.08 k | 1.99 k | --- | --- |
| Key: M = Mega-ohms (MΩ, $1 \times 10^6$ ohms), k = kilo-ohms (kΩ, $1 \times 10^3$ ohms). | | | | | | | | |

In every case, there is a clear difference in behaviour between the different specimens and most importantly, for the two fluids used. It should be noted that the same (logarithmic) scale has been used for all graphs, so that direct comparisons can be made, and any clear differences are in fact likely to be an order of magnitude or more.

For distilled water, MLSF001 and MLSF002 appear to have similar response times, in that the resistance of each takes approximately the same length of time to start to drop after the drops of fluid are added. This is about 30-90 seconds after fluid is added (t = 60-150 s). MLSF001 seems to have a more abrupt change in resistance when it responds, which is unexpected as the narrower conductive stripe separation of MLSF002 might have been expected to make it respond more quickly. Certainly this is the case for MLSF003, which responds almost instantaneously when the first drop of fluid is added (t ≈ 40 s). For each specimen, the two tests are reasonably similar in behaviour, with roughly the same shape of curve, and resistance values involved. For MLSF001 the change in resistance is by a factor of 53 and 7 for each of the tests, for MLSF002 it is by a factor of 32 and 26 for each of the tests, and for MLSF003 it is by a factor of 340 and 530.

The magnitude of the resistance drop is much greater for the saline solution, especially for the first of the two tests on each specimen. For the second test, the lowered starting resistance means that although the final resistance is close to that of the first test, the factor by which it has reduced is lower. The response time appears to be proportional to conductive stripe spacing, with MLSF003 faster than MLSF002, which was faster than MLSF001. Only MLSF002 responded noticeably faster with saline solution than with distilled water. For MLSF001 the change in resistance is by a factor of 1200 and 190 for each of the tests, for MLSF002 it is by a factor of 460 and 390 for each of the tests, and for MLSF003 it is by a factor of 6900 and 48.

It is clear from these results and from Figures 5 to 16, that it is easy to distinguish between distilled water and saline solution, even when the specimens have not had any special preparation to ensure that they are free of potentially conductive finishes. If the average of the two distilled water tests and the first saline solution test is compared, the difference in response is nearly two orders of magnitude for MLSF001 (30 c.f. 1200) and well over one order of magnitude for MLSF002 (29 c.f. 460) and MLSF003 (435 c.f. 6900).

Since urine and other body fluids contain substantial quantities of electrolyte, they may be considered analogous to the saline solution in the above tests. This means that the system as described could distinguish between wetting with water and wetting with body fluids such as urine.

In a further embodiment of the system, positive and negative tracks are provided on opposite sides of the fabric. In this case, electrical continuity between positive and negative tracks is provided as before, when wetting occurs. However, false positives do not occur as a result of wrinkles or folds in the fabric bringing positive and negative tracks together, as may possibly occur when positive and negative tracks occur on the same side of the fabric.

In a further embodiment of the system, a protective layer of fabric can be applied to cover the electrical tracks, to provide mechanical separation between tracks in the event of creasing or folding. This covering layer may be pervious to fluids, and so still allow electrical continuity between tracks by means of wetting.

In a further embodiment of the system, the conductive yarns are included in the system as a knitted fabric, rather than a woven fabric. This provides for stretchability of the material, which may be advantageous by causing less perturbation to the pressure-distributive properties of the mattress.

In a further embodiment, rows of staggered slits may be provided in the woven fabric so as to allow expansion of the material in a similar fashion to lattice pastry or expanded steel mesh. These slits may occur between tracks so as not to interfere with the electrical circuit. The slits would allow expansion and stretchability in the fabric, so causing less perturbation to the pressure-distributive properties.

In a further embodiment of the invention, soiling of the mattress core, identified as above by means of a recognisable change in electrical characteristics of the device, is immediately transmitted (by means of a radio frequency transmitter situated inside the mattress) to a remote receiver connected to a computer. This may be for example the nurses station, which records the time and location of the adverse event, so that suitable action may be taken. In this embodiment, local interrogation of the mattress is not required, nor is local memory storage within the mattress.

In a further embodiment, soiling of the mattress identified by the circuit prompts the microcontroller to effect an irreversible change in a visible indicator. The indicator may take the form of a permanently-changed bistable liquid crystal display, visible from the outside of the mattress cover with an appropriate clear window. Alternatively the visible indicator may comprise a combination of a small heating element inside the mattress cover, and an irreversible thermochromic strip in the corresponding place outside the mattress cover. Choosing an appropriate temperature threshold well above body temperature would eliminate false positives due to skin contact. Alternatively, the position of a mechanical flag can be activated irreversibly by means of a solenoid or other mechanical actuator. The position/condition of this flag may be determined by feeling through the mattress cover, without opening the cover. This flag may consist of a shaped memory alloy, which regains a predetermined shape irreversibly when subjected to an electrical stimulus.

Any of these embodiments obviate the need for electronic memory storage within the mattress, or for radio transmission, while still allowing inspection without opening the mattress cover, to determine whether historical leaks have occurred.

**Claims**

1. A mattress protector to fit over and shield a mattress or mattress core from body fluids, the protector comprising a shielding cover for the mattress or mattress core, the shielding cover having a layer that is impermeable to body fluids that is outermost in use, and a detector within the cover or below or in an under layer of the cover, the detector being below the impermeable layer of the shielding cover, to detect body fluid that has passed into or through the cover, the fluid having penetrated the impermeable layer of the shielding cover.

2. A mattress protector as claimed in Claim 1, wherein the cover is a transparent cover for a mattress with an inter-layer or under-layer having a dye that is activated by a body fluid to provide a visual indication of presence of the body fluid.

3. A mattress protector as claimed in Claim 2, wherein the dye is not reactive to water vapour.

4. A mattress protector as claimed in Claim 3, wherein the dye is specifically reactive to one or more organic compounds in urine, and/or other body fluids.

**5.** A mattress protector as claimed in any preceding claim, wherein the inter-layer or under-layer is absorbent.

**6.** A mattress protector as claimed in any preceding claim, wherein the inter-layer or under-layer is made from a stretchable material.

**7.** A mattress protector as claimed in Claim 1, 5, 6 or 7, wherein the detector comprises electrically conductive material in or associated with the inter-layer or under-layer whereby the detector responds to changes in electrical conductivity to detect body fluid that has passed through the cover

**8.** A mattress protector as claimed in Claim 7, wherein the detector comprises electrically conductive threads or fibres.

**9.** A mattress protector as claimed in Claim 8, wherein the electrically conductive threads or fibres are configured in rows or a matrix over the inter-layer or under-layer.

**10.** A mattress protector as claimed in Claim 8 or 9, wherein the electrically conductive threads form the warp or weft of the inter-layer or under layer.

**11.** A mattress protector as claimed in Claim 8, which further comprises a processor which monitors the electrical conductivity between neighbouring conductive threads so that in the event of a leak of ionic (electrically conductive) fluid into or through the mattress protector, a short circuit between threads will be detected and recorded.

**12.** A mattress protector as claimed in Claim 11, wherein the processor is a microprocessor integrated with the protector and adapted to be interrogated by an external device to determine if any leak events had occurred.

**13.** A mattress protector as claimed in Claim 12, wherein the microprocessor is within or below the cover and is adapted to be interrogated by an inductive link.

**14.** A mattress protector as claimed in any of Claims 7 to 13, wherein positive and negative tracks are provided on opposite sides of the fabric whereby wrinkles or folds in the fabric will not bring positive and negative tracks together.

**15.** A mattress protector as claimed in any of Claims 7 to 13, wherein a protective layer of fabric is applied covering the electrical tracks, to provide mechanical separation between tracks in the event of creasing or folding, the layer being pervious to fluids.

**16.** A mattress protector as claimed in any of Claims 8 to 15, wherein the conductive threads or fibres are included in the system as a knitted fabric, rather than a woven fabric for stretchability of the material.

**17.** A mattress protector as claimed in any of Claims 7 to 16, wherein the protector is of a woven fabric and slits are provided in the woven fabric so as to allow expansion of the material.

**18.** A mattress protector as claimed in any of Claims 7 to 17, wherein when the detector detects body fluid that has passed through the cover a signal is substantially immediately transmitted to a remote receiver connected to a computer.

**19.** A mattress protector as claimed in Claim 18, wherein the protector has a radio frequency transmitter situated inside the mattress to transmit the signal to the receiver.

**20.** A mattress protector as claimed in Claim 18 or 19, wherein the computer is programmed to record the time and location of the event, so that suitable action may be taken.

**21.** A mattress protector as claimed in any of Claims 7 to 20, wherein when the detector detects body fluid that has passed through the cover the circuit prompts a microcontroller to effect an irreversible change in a visible indicator.

**22.** A mattress protector as claimed in Claim 21, wherein the visible indicator is visible from the outside of the mattress cover via an appropriate clear window.

**23.** A mattress protector as claimed in Claim 21, wherein the visible indicator comprises a combination of a small heating element inside the mattress cover, and an irreversible thermochromic strip in the corresponding place outside the mattress cover.

24. A mattress protector as claimed in any of Claims 7 to 23, wherein the position of a mechanical flag can be activated irreversibly by means of a solenoid or other mechanical actuator and the position/condition of this flag may be determined by feeling through the mattress cover, without opening the cover.

25. A mattress protector as claimed in Claim 24, wherein the flag is formed of a shaped memory alloy, which regains a predetermined shape irreversibly when subjected to an electrical stimulus.

26. A mattress protector as claimed in any preceding claim in combination with a mattress.

27. A mattress core encased in a mattress protector of any of Claims 1 to 13.

**Patentansprüche**

1. Matratzenschutz, der über eine Matratze oder einen Matratzenkern passt und diese oder diesen gegen Körperflüssigkeiten abschirmt, wobei der Schutz eine Schutzabdeckung für die Matratze oder den Matratzenkern umfasst, wobei die Schutzabdeckung eine Schicht, die für Körperflüssigkeiten undurchdringlich ist und sich im Gebrauch auf der Außenseite befindet, und einen Detektor innerhalb der Schutzabdeckung oder unter der Schutzabdeckung oder in einer Unterschicht der Schutzabdeckung aufweist, wobei der Detektor unterhalb der undurchlässigen Schicht der Schutzabdeckung liegt, um Körperflüssigkeiten zu erfassen, die durch die Schutzabdeckung hindurch oder in diese gelangt sind, wobei die Flüssigkeit die undurchlässige Schicht der Schutzabdeckung durchdrungen hat.

2. Matratzenschutz nach Anspruch 1, bei dem die Schutzabdeckung eine transparente Abdeckung für eine Matratze mit einer Zwischenschicht oder Unterschicht ist, die einen Farbstoff aufweist, der durch eine Körperflüssigkeit aktiviert wird, um eine optische Anzeige des Vorhandenseins von Körperflüssigkeit zu liefern.

3. Matratzenschutz nach Anspruch 2, bei dem der Farbstoff nicht mit Wasserdampf reagiert.

4. Matratzenschutz nach Anspruch 3, bei dem der Farbstoff spezifisch auf eine oder mehrere organische Verbindungen in Urin und/oder anderen Körperflüssigkeiten reagiert.

5. Matratzenschutz nach einem der vorhergehenden Ansprüche, bei dem die Zwischenschicht oder die Unterschicht absorbierend ist.

6. Matratzenschutz nach einem der vorhergehenden Ansprüche, bei dem die Zwischenschicht oder die Unterschicht aus dehnbarem Material hergestellt ist.

7. Matratzenschutz nach Anspruch 1, 5, 6 oder 7, bei dem der Detektor elektrisch leitendes Material in der Zwischenschicht oder der Unterschicht oder in Verbindung mit diesen umfasst, wobei der Detektor auf Änderungen der elektrischen Leitfähigkeit anspricht, um Körperflüssigkeit zu erfassen, das durch die Abdeckung hindurch gelangt ist.

8. Matratzenschutz nach Anspruch 7, bei dem der Detektor elektrisch leitende Fäden oder Fasern umfasst.

9. Matratzenschutz nach Anspruch 8, bei dem die elektrisch leitenden Fäden oder Fasern in Reihen oder einer Matrix über der Zwischenschicht oder der Unterschicht konfiguriert sind.

10. Matratzenschutz nach Anspruch 8 oder 9, bei dem die elektrisch leitenden Fäden die Kette oder den Schuss der Zwischenschicht oder Unterschicht bilden.

11. Matratzenschutz nach Anspruch 8, der weiterhin einen Prozessor umfasst, der die elektrische Leitfähigkeit zwischen benachbarten leitenden Fäden überwacht, so dass im Fall des Leckens von ionischer (elektrisch leitender) Flüssigkeit in oder durch den Matratzenschutz hindurch ein Kurzschluss zwischen den Fäden erfasst und aufgezeichnet wird.

12. Matratzenschutz nach Anspruch 11, bei dem der Prozessor ein mit dem Schutz integrierter Mikroprozessor ist, der zum Abfragen durch ein externes Gerät ausgebildet ist, um festzustellen, ob irgendwelche Leckereignisse aufgetreten sind.

**13.** Matratzenschutz nach Anspruch 12, bei dem der Mikroprozessor innerhalb oder unter der Schutzabdeckung angeordnet ist und zum Abfragen durch eine induktive Verbindung ausgebildet ist.

**14.** Matratzenschutz nach einem der Ansprüche 7 bis 13, bei dem positive und negative Bahnen auf entgegengesetzten Seiten des Textilmaterials vorgesehen sind, so dass Knitter oder Falten in dem Gewebe positive und negative Bahnen nicht zusammenführen.

**15.** Matratzenschutz nach einem der Ansprüche 7 bis 13, bei dem eine Schutzschicht aus textilmaterial, die die elektrischen Bahnen abdeckt, angebracht ist, um eine mechanische Trennung zwischen den Bahnen im Fall eines Knitterns oder Faltens zu schaffen, wobei die Schicht für Flüssigkeiten durchdringbar ist.

**16.** Matratzenschutz nach einem der Ansprüche 8 bis 15, bei dem die leitenden Fäden oder Fasern in dem System als ein gewirktes Textilmaterial statt als ein gewebtes Textilmaterial für eine Dehnbarkeit des Materials eingefügt sind.

**17.** Matratzenschutz nach einem der Ansprüche 7 bis 16, bei dem der Schutz ein gewebtes Textilmaterial ist und Schlitze in dem gewebten Textilmaterial vorgesehen sind, um eine Dehnung des Materials zu ermöglichen.

**18.** Matratzenschutz nach einem der Ansprüche 7 bis 17, bei dem ein Signal im Wesentlichen unmittelbar an einen entfernt angeordneten, mit einem Computer verbundenen Empfänger übertragen wird, wenn der Detektor feststellt, dass Flüssigkeit durch die Schutzabdeckung hindurch gelangt ist.

**19.** Matratzenschutz nach Anspruch 18, bei dem die Schutzabdeckung einen Hochfrequenzsender aufweist, der auf der Innenseite der Matratze angeordnet ist, um das Signal an den Empfänger zu senden.

**20.** Matratzenschutz nach Anspruch 18 oder 19, bei dem der Computer zur Aufzeichnung der Zeit und des Ortes des Ereignisses programmiert ist, so dass eine geeignete Maßnahme getroffen werden kann.

**21.** Matratzenschutz nach einem der Ansprüche 7 bis 20, bei dem die Schaltung einen Mikrocontroller anweist, eine nicht umkehrbare Änderung einer optischen Anzeige zu bewirken, wenn der Detektor Körperflüssigkeit erfasst, die durch die Schutzabdeckung hindurch gelangt ist.

**22.** Matratzenschutz nach Anspruch 21, bei der die optische Anzeige von der Außenseite der Matratzenabdeckung über ein geeignetes klares Fenster sichtbar ist.

**23.** Matratzenschutz nach Anspruch 21, bei dem die optische Anzeige eine Kombination eines kleinen Heizelementes innerhalb der Matratzenabdeckung und einen nichtumkehrbaren thermochromen Streifen an der entsprechenden Stelle auf der Außenseite der Matratzenabdeckung umfasst.

**24.** Matratzenschutz nach einem der Ansprüche 7 bis 23, bei dem die Position einer mechanischen Flagge nicht umkehrbar mit Hilfe einer Magnetspule oder einer anderen mechanischen Betätigungseinrichtung aktiviert werden kann und die Position/der Zustand dieser Flagge durch Fühlen durch die Matratzenabdeckung ohne Öffnen der Abdeckung gefühlt werden kann.

**25.** Matratzenschutz nach Anspruch 24, bei dem die Flagge aus einer geformten Legierung mit Formgedächtnis gebildet ist, die eine vorgegebene Form in nicht umkehrbarer Weise annimmt, wenn sie einer elektrischen Stimulierung unterworfen wird.

**26.** Matratzenschutz nach einem der vorhergehenden Ansprüche, in Kombination mit einer Matratze.

**27.** Matratzenkern, der von einem Matratzenschutz nach einem der Ansprüche 1 bis 13 umgeben ist.


**Revendications**

**1.** Dispositif de protection de matelas destiné à s'adapter sur et à protéger un matelas ou une partie centrale de matelas contre les fluides corporels, le dispositif de protection comprenant une housse de protection pour le matelas ou la partie centrale de matelas, la housse de protection ayant une couche qui est imperméable aux fluides corporels qui est située le plus à l'extérieur à l'usage, et un détecteur à l'intérieur de la housse ou au-dessous ou dans une sous-couche de la housse, le détecteur étant au-dessous de la couche imperméable de la housse de protection, pour détecter le fluide corporel qui est passé dans ou à travers la housse, le fluide ayant pénétré dans la couche imperméable de la housse de protection.

2. Dispositif de protection de matelas selon la revendication 1, dans lequel la housse est une housse transparente pour un matelas avec une couche intermédiaire ou une sous-couche ayant un colorant qui est activé par un fluide corporel pour fournir une indication visuelle de la présence du fluide corporel.

3. Dispositif de protection de matelas selon la revendication 2, dans lequel le colorant n'est pas réactif à la vapeur d'eau.

4. Dispositif de protection de matelas selon la revendication 3, dans lequel le colorant est spécifiquement réactif à un ou plusieurs composés organiques présents dans l'urine et/ou d'autres fluides corporels.

5. Dispositif de protection de matelas selon l'une quelconque des revendications précédentes, dans lequel la couche intermédiaire ou la sous-couche est absorbante.

6. Dispositif de protection de matelas selon l'une quelconque des revendications précédentes, dans lequel la couche intermédiaire ou la sous-couche est réalisée avec un matériau extensible.

7. Dispositif de protection de matelas selon la revendication 1, 5, 6 ou 7, dans lequel le détecteur comprend un matériau électriquement conducteur dans ou associé à la couche intermédiaire ou la sous-couche moyennant quoi le détecteur répond aux changements de conductivité électrique pour détecter le fluide corporel qui est passé à travers la housse.

8. Dispositif de protection de matelas selon la revendication 7, dans lequel le détecteur comprend des fils ou fibres électriquement conducteurs.

9. Dispositif de protection de matelas selon la revendication 8, dans lequel les fils ou fibres électriquement conducteurs sont configurés en rangées ou en matrice sur la couche intermédiaire ou la sous-couche.

10. Dispositif de protection de matelas selon la revendication 8 ou 9, dans lequel les fils électriquement conducteurs forment la chaîne ou la trame de la couche intermédiaire ou de la sous-couche.

11. Dispositif de protection de matelas selon la revendication 8, comprenant en outre un processeur qui surveille la conductivité électrique entre les fils conducteurs voisins de sorte que dans le cas d'une fuite de fluide ionique (électriquement conductrice) dans ou à travers le dispositif de protection de matelas, on détecte et on enregistre un court-circuit entre les fils.

12. Dispositif de protection de matelas selon la revendication 11, dans lequel le processeur est un microprocesseur intégré avec le dispositif de protection et adapté pour être interrogé par un dispositif externe afin de déterminer si l'un quelconque des évènements de fuite s'est produit.

13. Dispositif de protection de matelas selon la revendication 12, dans lequel le microprocesseur est à l'intérieur ou au-dessous de la housse et est adapté pour être interrogé par un lien inductif.

14. Dispositif de protection de matelas selon l'une quelconque des revendications 7 à 13, dans lequel les cheminements positif et négatif sont prévus sur les côtés opposés du tissu, moyennant quoi des faux plis ou des plis dans le tissu ne mettent pas les cheminements positif et négatif en contact.

15. Dispositif de protection de matelas selon l'une quelconque des revendications 7 à 13, dans lequel une couche de protection de tissu est appliquée en recouvrant les cheminements électriques, afin de fournir la séparation mécanique entre les cheminements dans le cas de froissement ou de pliage, la couche étant perméable aux fluides.

16. Dispositif de protection de matelas selon l'une quelconque des revendications 8 à 15, dans lequel les fils ou fibres conducteurs sont compris dans le système tel qu'un tissu tricoté, plutôt qu'un tissu tissé pour l'extensibilité du matériau.

17. Dispositif de protection de matelas selon l'une quelconque des revendications 7 à 16, dans lequel le dispositif de protection est en tissu tissé et des fentes sont prévues dans le tissu tissé afin de permettre l'expansion du matériau.

**18.** Dispositif de protection de matelas selon l'une quelconque des revendications 7 à 17, dans lequel lorsque le détecteur détecte le fluide corporel qui est passé à travers la housse, un signal est transmis pratiquement immédiatement à un récepteur à distance raccordé à un ordinateur.

**19.** Dispositif de protection de matelas selon la revendication 18, dans lequel le dispositif de protection a un émetteur à radiofréquence situé à l'intérieur du matelas pour transmettre le signal au récepteur.

**20.** Dispositif de protection de matelas selon la revendication 18 ou 19, dans lequel l'ordinateur est programmé pour enregistrer le moment et le lieu de l'évènement, de sorte que la mesure appropriée peut être prise.

**21.** Dispositif de protection de matelas selon l'une quelconque des revendications 7 à 20, dans lequel lorsque le détecteur détecte le fluide corporel qui est passé à travers la housse, le circuit incite un microcontrôleur à effectuer un changement irréversible dans un indicateur visible.

**22.** Dispositif de protection de matelas selon la revendication 21, dans lequel l'indicateur visible est visible depuis l'extérieur de la housse de matelas via une fenêtre transparente appropriée.

**23.** Dispositif de protection de matelas selon la revendication 21, dans lequel l'indicateur visible comprend une combinaison d'un petit élément chauffant à l'intérieur de la housse de matelas, et d'une bande thermochromatique irréversible dans le lieu correspondant à l'extérieur de la housse de matelas.

**24.** Dispositif de protection de matelas selon l'une quelconque des revendications 7 à 23, dans lequel la position d'un drapeau mécanique peut être activée de manière irréversible au moyen d'un solénoïde ou d'un autre actionneur mécanique et la position / condition de ce drapeau peut être déterminée par sensation à travers la housse de matelas, sans ouvrir la housse.

**25.** Dispositif de protection de matelas selon la revendication 24, dans lequel le drapeau est formé avec un alliage à mémoire de forme, qui retrouve une forme prédéterminée de manière irréversible lorsqu'il est soumis à un stimulus électrique.

**26.** Dispositif de protection de matelas selon l'une quelconque des revendications précédentes, en combinaison avec un matelas.

**27.** Partie centrale de matelas enfermée dans un dispositif de protection de matelas selon l'une quelconque des revendications 1 à 13.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6341393 B, Votel